(19) Europäisches Patentamt

European Patent Office

Office européen des brevets

(11) Veröffentlichungsnummer: **0 373 488 B1**

# (12) EUROPÄISCHE PATENTSCHRIFT

(45) Veröffentlichungstag der Patentschrift: **19.05.93**

(21) Anmeldenummer: **89122437.0**

(22) Anmeldetag: **05.12.89**

(51) Int. Cl.5: **C07F 13/00**, C07C 6/04, B01J 31/26, C07B 37/00

(54) Organische Derivate von Rheniumoxiden sowie ihre Herstellung und Verwendung zur Metathese von Olefinen.

(30) Priorität: **10.12.88 DE 3841733**
**31.01.89 DE 3902787**

(43) Veröffentlichungstag der Anmeldung:
**20.06.90 Patentblatt 90/25**

(45) Bekanntmachung des Hinweises auf die
Patenterteilung:
**19.05.93 Patentblatt 93/20**

(84) Benannte Vertragsstaaten:
**BE CH DE FR GB IT LI NL**

(56) Entgegenhaltungen:

**ANGEWANDTE CHEMIE, International edition
in English, Band 27, Nr. 3, 1988, Seiten
394–396; W.A. HERRMANN et al.:
"Methylrhenium oxides: Synthesis from dirhenium heptoxide and catalytic activity in
olefin metathesis"**

**J. BUCKINGHAM: "Dictionary of Organometallic Compounds", Band 2, 1984, Seite 1637,
Chapman and Hall, London, GB**

**ANGEWANDTE CHEMIE, Band 27, Nr. 10, Ok‐**

**tober 1988, Seiten 1297–1313, VCH Verlagsgesellschaft mbH, Weinheim, DE; W.A. HER‐
MANN: "High oxidation state organometallic
chemistry, a challenge – the example of
rhenium"**

(73) Patentinhaber: **HOECHST AKTIENGESELL‐
SCHAFT
Postfach 80 03 20
W–6230 Frankfurt am Main 80(DE)**

(72) Erfinder: **Herrmann, Wolfgang Anton,
Prof.–Dr.
Waldweg 10
W–8051 Giggenhausen(DE)**
Erfinder: **Wagner, Werner
Schlierseestrasse 29
W–8000 München 90(DE)**
Erfinder: **Volkhardt, Ursula
Heiliggeistgasse 4
W–8050 Freising(DE)**

EP 0 373 488 B1

**Beschreibung**

Die vorliegende Erfindung bezieht sich auf organische Derivate von Rheniumoxiden, ihre Herstellung und Verwendung zur Metathese von nicht−funktionalisierten wie auch funktionalisierten Olefinen.

Der Begriff Metathese bezeichnet bekanntlich die Spaltung von olefinischen Verbindungen in der Weise, daß die Doppelbindung auseinandergebrochen wird und die beiden Bruchstücke sich zu neuen Verbindungen vereinigen. Zur Metathese gehört im weiteren Sinne auch die Ringöffnungspolymerisation von Cycloolefinen. Durch die Erfindung wird erstmals eine in Substanz isolierbare Organorhenium−Verbindung definierter Struktur als Katalysator erhalten.

Es ist bekannt, daß Verbindungen des Elements Rhenium katalytische Aktivitäten in der Olefin−Metathese aufweisen, und zwar nur bei der Metathese nicht−funktionalisierter Olefine. Nur wenn man solchen Rhenium−Katalysatoren sogenannte Cokatalysatoren, insbesondere Tetraalkylzinn−Verbindungen zusetzt, gelingt auch die Metathese funktionalisierter, d. h. Heterofunktionen aufweisender Olefine (K. J. Ivin: Olefin Metathesis, Academic Press, London 1983, 32 − 34). So wurde die Metathese von olefinisch ungesättigten Carbonsäureestern, von Allylhalogeniden usw. an Mehrkomponenten−Katalysatoren wie $WCl_6/Sn(CH_3)_4$ oder $Re_2O_7/Sn(CH_3)_4/Al_2O_3$ beschrieben. Das letztgenannte System (R. A. Fridman et al., Dokl. Akad. Nauk. SSSR 234, (1977), 1354 − 57; R. H. A. Bosma et al., J. Organometal. Chem. 225 (1983) 159 − 171; X. Xiaoding et al., J. Chem. Soc. Chem. Commun. 1985, 631 − 633; G. C. N. van den Aardweg, ibid. 1983, 262 − 263) ist das einzige rheniumhaltige, welches olefinisch ungesättigte organische Halogenide, Ester usw. zu metathetisieren imstande ist. Über die wirksame Katalysatorspezies hat man bisher keine Kenntnis. Praktische Nachteile der bisher verwendeten rheniumhaltigen Katalysatorsysteme sind

a) die Notwendigkeit, bei der Metathese funktionalisierter Olefine hochtoxische Tetraalkylzinn−Verbindungen als Aktivatoren mitzuverwenden (J. E. Cremer, Biochem. J. 68 (1958) 685 − 688; W. N. Aldridge et al., The Lancet, Sept. 26, 1981, S. 692/3),

b) geringe, für die industrielle Nutzung nicht tragbare Katalysatoraktivitäten,

c) relativ eng begrenzte Anwendungsbreite, d. h. die katalytische Wirkung tritt nur bei wenig bzw. nicht funktionalisierten Olefinen ein.

Es bestand daher die Aufgabe, ein möglichst leicht zugängliches, einfach handhabbares, lagerfähiges, ungiftiges und wirksames Katalysatorsystem zu finden, das auch bei der Metathese funktionalisierter Olefine ohne jeden Aktivator auskommt. Den Produkten kommt für die industrielle Weiterverarbeitung großes Interesse zu.

Einige sogenannte "Organorheniumoxide", z. B. $CH_3ReO_3$, $(\eta^5-C_5(CH_3)_5)ReO_3$ ( = Pentamethylcyclopentadienyl−Rheniumtrioxid) sind als Katalysatorkomponenten der homogen−katalysierten Olefin−Metathese bekannt (W. A. Herrmann et al., Angew. Chem. 100 (1988) 420 − 422, Übersetzung in Angew. Chem. Int. Ed. Engl. 27 (1988) 394 − 396). So ist aus dieser Arbeit bekannt, daß Methylrheni−umoxid $CH_3ReO_3$ in Gegenwart von $AlCl_3$ und Tetramethylzinn bereits bei Raumtemperatur die Metathese offenkettiger Olefine und auch die katalytische Ringöffnungspolymerisation von Cycloolefinen zu Polyalkenameren bewirkt. Aus der gleichen Veröffentlichung ist auch bekannt, daß das Methylrheniumoxid bei der Behandlung mit Dimethylzink zu einem sublimierbaren, zitronengelben Methylrheniumoxid der Formel $(CH_3)_4Re_2O_4$ methyliert wird, also zu einer Verbindung mit 6−wertigem Rhenium. Auch diese Verbindung katalysiert in Gegenwart von Tetramethylzinn und Aluminiumchlorid die metathetische Ringöffnungspolymerisation von Cyclopenten.

Aus der genannten Veröffentlichung ist schließlich bekannt, daß das $(CH_3)_4Re_2O_4$ durch weitere Methylierung mit Dimethylzink zu $(CH_3)_6Re_2O_3$ umgesetzt wird, also ebenfalls zu einer Verbindung mit 6−wertigem Rhenium. Diese Verbindung ist nach der genannten Literaturstelle in der Lage, die Ringöffnungspolymerisation von Cyclopenten selbst dann zu katalysieren, wenn nur $AlCl_3$, nicht aber der sonst noch notwendige Cokatalysator $Sn(CH_3)_4$ zugegen ist. Es war bisher also stets die Mitverwendung einer in organischen Lösungsmitteln zumindest teilweise löslichen Lewis−Säure als Aktivator nötig, vorzugsweise Aluminium(III)−chlorid, und in den meisten Fällen auch die Mitverwendung von toxischen Zinntetraalkylen (z. B. $Sn(CH_3)_4$) als weiteren Aktivatoren. Selbst dann sind die so erhältlichen Katalysatorsysteme bezüglich der Metathese funktionalisierter Olefine völlig inaktiv.

Es wurde nun überraschend gefunden, daß auf oxidische Trägermaterialien, insbesondere Aluminiumoxid−Träger, aufgebrachtes Methylrheniumtrioxid $CH_3ReO_3$ und verwandte Verbindungen der Formel $R^1_aRe_bO_c$ (I) als Metathesekatalysator geeignet sind. Dabei entfällt erstmals die aus vielen Gründen nachteilige Verwendung zusätzlicher Aktivatorsubstanzen ("Cokatalysatoren").

Gegenstand der Erfindung ist somit die Verwendung von solchen Verbindungen des Rheniums, die die allgemeine Formel $R^1_aRe_bO_c$ (I) haben, worin a 1 bis 6, b 1 bis 4 und c 1 bis 14 sind und die Summe von a, b und c so ist, daß sie der 5− bis 7−Wertigkeit des Rheniums gerecht wird, mit der Maßgabe, daß c nicht größer als 3•b ist, und die auf oxydische Trägermaterialien aufgebracht sind, als Heterogenkatalysatoren für die Metathese. Dabei bedeutet $R^1$ eine organische Gruppe, die über ein Kohlenstoffatom, an das noch wenigstens ein Wasserstoffatom gebunden ist, an das Metall Rhenium gebunden ist, und zwar Alkylreste mit 1 bis 9 C−Atomen, Cycloalkyl mit 5 bis 10 C−Atomen, wie Cyclohexyl oder 1−Norbornyl, oder Aralkyl mit 7 bis 9 C−Atomen, wie Benzyl, vorzugsweise aber Methyl. Die Begriffe Alkyl und Cycloalkyl beinhalten naturgemäß, daß diese Gruppen keine Mehrfachbindungen enthalten. $R^1$ kann wenigstens teilweise fluoriert sein. Jedoch kommt in den Verbindungen aus sterischen Gründen die Anwesenheit von mehr als drei Gruppen mit mehr als 6 C−Atomen je Rheniumatom nicht in Betracht; zweckmäßig enthalten die Verbindungen nur höchstens eine solche Gruppe. Der Begriff der Metathese schließt hierbei die Ringöffnungspolymerisation von Cycloolefinen ein.

Von den Verbindungen der vorgenannten Formel sind das $CH_3ReO_3$, das $(CH_3)_6Re_2O_3$, das $(CH_3)−_4Re_2O_4$, $(CH_3)_4ReO$, das $(CH_3)_3ReO_2$, das $[(CH_3)_3C−CH_2]_3ReO_2$, das $[(CH_3)_3\ C−CH_2]_4Re_2O_4$ und das Pentamethylcyclopentadienyl−Rheniumtrioxid zwar bekannt, jedoch war die katalytische Wirksamkeit dieser Verbindungen ohne Mitverwendung von Aktivatoren, also die Eignung als alleiniger Katalysator, nicht bekannt und ebensowenig zu erwarten wie die der ganzen Verbindungsklasse. Diese katalytische Wirkung überrascht um so mehr, als das isostrukturelle (Trimethylstannoxy)−rheniumtrioxid $[(CH_3)_3SnO]ReO_3$ katalytisch unwirksam ist. Dies zeigt die Bedeutung der an Rhenium gebundenen Alkylgruppen für die katalytische Wirksamkeit der erfindungsgemäßen Verbindungen.

Gegenstand der Erfindung sind daher auch die Verbindungen der vorgenannten Formel I mit Ausnahme des $CH_3ReO_3$, des $(CH_3)_6Re_2O_3$, des $(CH_3)_4Re_2O_4$, des $(CH_3)_4ReO$, des $(CH_3)_3ReO_2$, des $[(CH_3)_3C−CH_2]_3ReO_2$, des $[(CH_3)_3C−CH_2]_4Re_2O_4$ und des Pentamethylcyclopentadienyl−Rheniumtrioxids.

Die erfindungsgemäßen Verbindungen sind synthetisch auf sehr einfache Weise aus $Re_2O_7$ mit Hilfe üblicher Alkylierungsmittel herstellbar. Z. B. setzt man Dirheniumheptoxid in einem wasserfreien, gegenüber den Rheniumverbindungen inerten Lösungsmittel bei einer Temperatur von 0 bis 60, vorzugsweise 10 bis 40°C, mit einer Lösung von $R^1_2Zn$ um, wobei $R^1$ die oben angegebene Bedeutung hat, und entfernt die flüchtigen Anteile. Die erfindungsgemäßen Verbindungen sind völlig unempfindlich gegenüber Luft und Feuchtigkeit. Die auf oxidische Trägermaterialien aufgebrachten Rheniumverbindungen sind hochaktive Katalysatoren für die Metathese nicht−funktionalisierter wie auch funktionalisierter Olefine des Typs $YCZ=CZ−(CX_2)_nR^2$ (II), wobei n eine ganze Zahl von 1 bis 28, X H oder F ist, Y = H oder Alkyl mit 1 bis 10 C−Atomen ist und Z H oder einen nicht−aromatischen Kohlenwasserstoffrest mit 1 bis 6 C−Atomen darstellt, der z. B. Cyclohexyl ist, vorzugsweise aber offenkettiges Alkyl mit 1 bis 4 C−Atomen ist, und der Substituent $R^2$ H, Alkyl, Halogen, $COOR^3$ oder $OR^4$ ist, worin $R^3$ Alkyl oder Aryl und $R^4$ Alkyl, Aryl oder Trialkylsilyl $R^5_3Si$ ist. Die Alkylgruppen in $R^5$ enthalten 1 bis 5, vorzugsweise 1 bis 3 C−Atome. In $R^3$ und $R^4$ steht Alkyl für 1 bis 15, vorzugsweise 1 bis 6 Kohlenstoffatome und Aryl für Phenyl, das am Ring noch ein bis drei Substituenten enthalten kann, wie Halogen, z. B. Fluor, Chlor oder Brom, $NO_2$, $NR^6R^7$, $OR^8$ und/oder Alkyl. Die Reste $R^6$, $R^7$ und $R^8$ sind gleich oder verschieden und können Wasserstoff oder Alkyl mit 1 bis 4 C−Atomen sein. Die Gruppen YCZ und $CZ−(CH_2)_nR^2$ müssen jedoch verschieden sein. $R^2$ als Halogen kann Fluor, Chlor, Brom oder Jod sein. Wenn Z Wasserstoff und $R^2$ Halogen ist, ist dieses bevorzugt Brom. Verbindungen, in denen wenigstens ein X = F ist, sind z. B. 4−(Perfluor−n−hexyl)−buten(1) der Formel $(n−C_6F_{13})−CH_2−CH_2−CH=CH_2$ und Perfluorpropen $C_3F_6$. Die Gliederzahl n variiert vorzugsweise im Bereich von 1 bis 12 und insbesondere bis 8.

Die neuartigen Katalysatoren sind also nicht nur bei Olefinen mit Z = H, sondern auch bei der Metathese teilweise fluorierter Olefine wirksam. Sie eignen sich auch für die Metathese innenständiger, funktionalisierter Olefine der Formel $R^9CH=CH−(CH_2)_nR^{10}$ (IV), worin $R^9$ ein verzweigter oder zweckmäßig unverzweigter Alkylrest mit 1 bis 12 C−Atomen, $R^{10}$ ein Carboxyalkylrest ist, worin der Alkylrest zweckmäßig 1 bis 4 C−Atome hat und n eine ganze Zahl von 1 bis 10 darstellt. Beispielhaft sei Methyloleat genannt ($R^9$ = n−Octyl, $R^{10}$ = $CO_2CH_3$, n = 7).

Als Trägermaterialien eignen sich besonders gut $SiO_2/Al_2O_3$ (z. B. im Gewichtsverhältnis 87 : 13) und $Al_2O_3$, das je nach Vorbehandlung sauer, neutral oder basisch sein kann. Die Aktivität dieser Katalysatoren kann erheblich gesteigert werden, wenn die Rheniumverbindungen auf einen ausgeglühten, d. h. möglichst wasserfreien Träger wie Kiesel−/Aluminiumoxid aufgebracht werden. Enthält nämlich das Trägermaterial größere Feuchtigkeitsmengen, so verringert sich die Aktivität, weil dann die an Rhenium gebundene Alkylgruppe durch das Wasser teilweise als Alkan abgespalten wird.

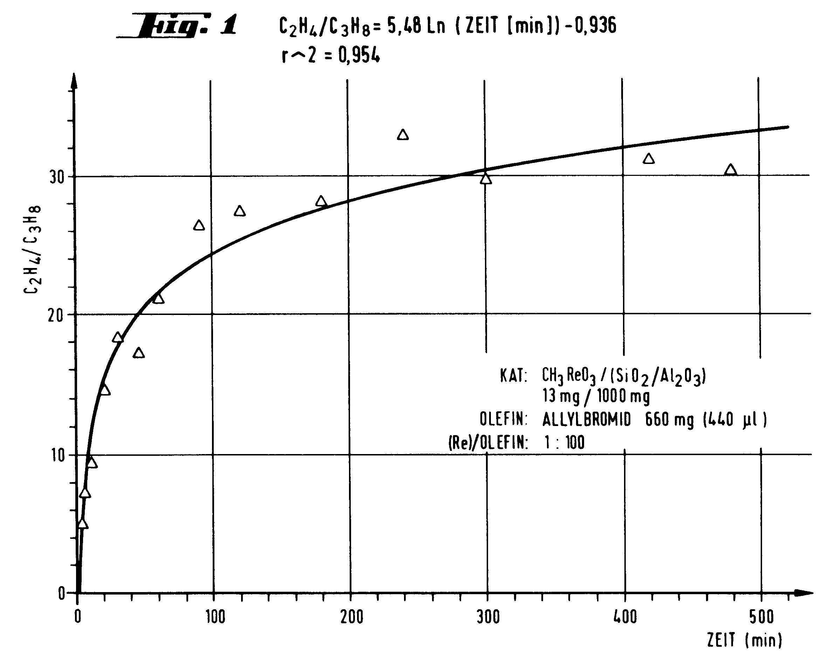

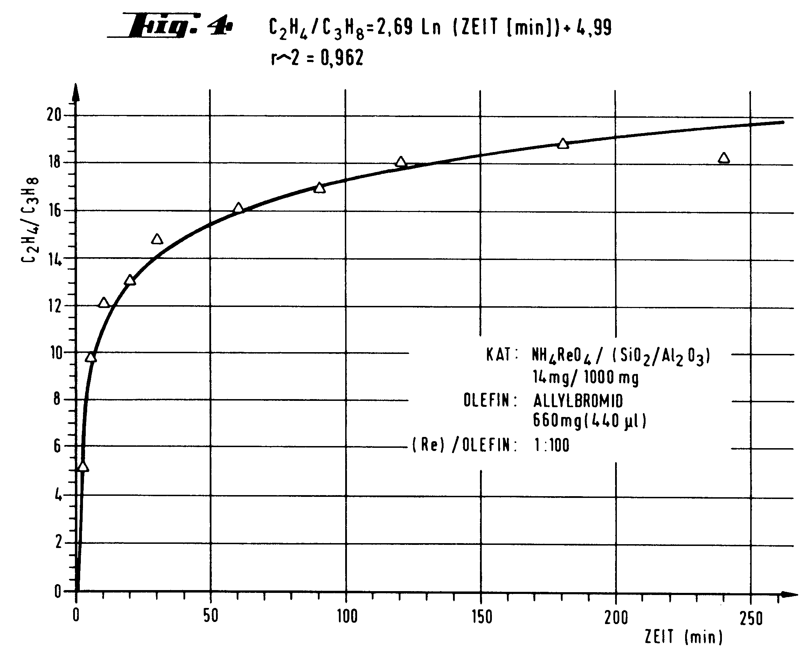

Die katalytische Aktivität der erfindungsgemäßen Katalysatoren für die Metathese von Olefinen ist höher als bei den bisher beschriebenen rheniumhaltigen Katalysatorsystemen. Als beispielhafter Beleg dienen die Ansätze gemäß Fig. 1 und Fig. 4. Das System $CH_3ReO_3/SiO_2/Al_2O_3$ (Fig. 1) metathetisiert Allylbromid bereits in 20 min zu Q über 25, wobei der

$$\text{Quotient } Q = \frac{\text{Peakfläche Äthylen}}{\text{Peakfläche Propan}}$$

mit Propan als vorgegebenem Standard gebildet und gegen die Zeit aufgetragen wird. Der Kurvenverlauf gibt Aufschluß über die Aktivität des jeweiligen Katalysatorsystems. Der literaturbekannte Standard – Katalysator $NH_4[ReO_4]/SiO_2/Al_2O_3$ (Fig. 4) wirkt dagegen nur in Gegenwart von $Sn(CH_3)_4$, und auch dann erst in 180 min, in vergleichbarem Maße. Das Symbol "r^2" ist ein Maß für die Übereinstimmung des rechnerisch ermittelten Kurvenverlaufs mit den experimentellen Werten.

Die bisher in der Olefin – Metathese technisch verwendeten rheniumhaltigen Katalysatoren werden in der Regel so hergestellt (S. Warwel et al., Chem. – Ztg. 107 (1983) 115 – 120), daß handelsübliches Ammoniumperrhenat $NH_4[ReO_4]$ als Dioxan/Wasser – Lösung mit dem Trägermaterial (in der Regel Alumi – niumoxid) behandelt wird. Die entstandene Suspension wird dann im Vakuum der Wasserstrahlpumpe zur völligen Trockne gebracht; das trockene Material wird dann bei 550°C zunächst etwa 2 h im Sauerstoff – strom und dann etwa 2 h im Stickstoffstrom erhitzt. Erst das so erhaltene, auf Raumtemperatur abgekühlte Material wird für die Katalysezwecke eingesetzt.

Für das erfindungsgemäße Verfahren hingegen kann man auf diese zeit – und energieaufwendigen Arbeitsschritte verzichten, wodurch auch besser reproduzierbare Katalysatoren erhalten werden. Die kata – lytisch wirkende Rheniumverbindung wird für das vorliegende Verfahren vorteilhaft bei Raumtemperatur aus einem Lösungsmittel, vorzugsweise einer Dichlormethan – Lösung, auf den Katalysatorträger, vorteilhaft Kieselgel/Aluminiumoxid, aufgebracht, wobei lediglich der Katalysatorträger vor seiner Verwendung 2 h bei 550 bis 800°C im Stickstoffstrom von Feuchtigkeit befreit wird, damit das Katalysatorsystem nachher seine volle Aktivität entfaltet.

Bei der Metathese mit den erfindungsgemäßen Katalysatoren ist darauf zu achten, daß Luft und Feuchtigkeit ausgeschlossen werden. Auch die eingesetzten Olefine sind vor ihrer Verwendung zweckmäßig gut zu trocknen. Die Metathese wird im allgemeinen bei Atmosphärendruck und einer Temperatur von 0 bis 60°C zweckmäßig 10 bis 30°C durchgeführt. Die Möglichkeit, bei solch milden Reaktionsbedingungen zu arbeiten, ist ein besonderer Vorteil des erfindungsgemäßen Verfahrens. Es ist aber auch möglich, noch höhere Temperaturen, z. B. bis 100°C anzuwenden oder bei Über – oder Unterdruck zu arbeiten. Jedoch sind hiermit gewöhnlich keine Vorteile verbunden.

In den Charakterisierungen einzelner Stoffe in den folgenden Beispielen bedeutet "sst" sehr stark, "st" stark, "br" breit und "EI – MS" Electron – Impact – Massenspektrum.

**Beispiele 1 – 5 Metathese mit $CH_3ReO_3$**

In den folgenden Versuchen wurden die eingesetzten Olefine vor ihrer Verwendung über $CaH_2$ getrocknet und destilliert. Als Katalysator wurde dabei Methylrheniumtrioxyd $CH_3ReO_3$ verwendet, das nach einem präparativ einfachen, das giftige Tetramethylzinn, $Sn(CH_3)_4$,vermeidenden Verfahren wie folgt her – gestellt worden war:

Eine Lösung von 4,84 g (10 mmol) Dirheniumheptoxid $Re_2O_7$ (Fa. Degussa, 76,9 % Re) in 100 ml eines wasserfreien Lösungsmittels wie Tetrahydrofuran wurde bei Raumtemperatur mit 20 ml einer 0,5 – molaren Lösung von Dimethylzink $Zn(CH_3)_2$ in Tetrahydrofuran binnen 10 min tropfenweise versetzt und dann noch 60 min bei Raumtemperatur gerührt. Die flüchtigen Anteile wurden dann im Vakuum einer Ölpumpe in eine Kühlfalle abgezogen. Der Rückstand wurde in einem Hochvakuum bei 40 – 55°C an einem wassergekühlten Sublimationsfinger sublimiert. Dabei erhielt man 3,89 g (78 % d. Theorie) farblose, meist nadelförmige Kristalle des Katalysators $CH_3ReO_3$. Die Synthese gelingt auch mit nicht – sublimiertem $Re_2O_7$, doch sind die Ausbeuten dann geringer. Die Substanz ist folgendermaßen charakterisiert Schmelzpunkt 106°C. – IR ($cm^{-1}$, KBr): 1002 sst, 950 sst,br[v(Re = O)]. – ¹H – NMR ($CDCl_3$, 28°C): $\delta(CH_3)$ = 2,61 [Singulett]. – ¹³C – NMR ($CDCl_3$, 28°C): $\delta(CH_3)$ = 19,03 [Quartett, $^1J(C,H)$ = 138 Hz]. – ¹⁷O – NMR ($CDCl_3$, 28°C):$\delta(O)$ = 829 ppm. – EI – MS : m/z = 250 (Molekül – Ion, mit korrektem Isotopenmuster $^{185}Re/^{187}Re$). – Die Substanz kann bei Raumtemperatur unzersetzt aufbewahrt werden. – Elementaranalyse: Ber. für $CH_3O_3Re$ (249,21): C 4,82, H 1,20, O 19,26, Re 74,72; Gef.: C 4,84, H 1,19, O 19,30, Re 74,78.

In einem 30 ml – Reaktionsgefäß mit Septum, Rückflußkühler und Quecksilberüberdruckventil wurde unter Rühren eine Lösung von 13 mg (0,052 mmol) Methylrheniumtrioxid $CH_3ReO_3$ in 0,5 ml Dichlormethan in eine Suspension von 1 000 mg Katalysatorträger [$SiO_2/Al_2O_3$, (87 : 13), Korngröße unter 15 μm (Präparat

Nr. 14−7150 der Fa. Strem Chemicals, Newburyport/ Mass. 01950 (USA), 2 h auf 550°C erhitzt] in 10 ml Dichlormethan (über Calciumhydrid getrocknet und unter Stickstoffatmosphäre aufbewahrt) eingetragen. Der Kolbeninhalt wurde zum Sieden erhitzt. Nach Einstellen des thermischen Gleichgewichts wurden 5,2 mmol Olefin mit einer Spritze durch das Septum injiziert. Zur Isolierung des Produkts wurde nach mehrstündigem Kochen unter Rückfluß (Tab. 1) der Kontakt auf einer Fritte abgesaugt und zweimal mit je 10 ml Dichlormethan ausgewaschen. Das Lösungsmittel wurde im Vakuum einer Ölpumpe abgezogen, das Produkt ausgewogen und durch einen Gaschromatographen mit angekoppeltem Massenspektrometer auf Reinheit überprüft.

**Tabelle 1: Metathese funktionalisierter α-Olefine vom Typ II unter Abspaltung von Äthylen**

| Beispiel/Olefin (*) | Zeit (h) | Menge (mg) | Auswaage (mg) | Reinheit (%) | Ausbeute (%) |
|---|---|---|---|---|---|
| 1) Allylbromid | 4 | 620 | 100 | 100 | 14 |
| 2) Undecensäureäthylester | 20 | 1120 | 1120 | 64 | 64 |
| 3) Allylisopropyläther | 4 | 520 | 220 | 70 | 30 |
| 4) Allyläthyläther | 5 | 448 | 260 | 50 | 39 |
| 5) Allyltrimethyl-silyläther | 24 | 680 | 530 | 88 | 69 |

(*) Metatheseprodukte:

    1) 1,4-Dibrombuten(2).

    2) Eicosen(10)-ω,ω'-dicarbonsäurediäthylester.

    3) 1,4-Bis(isopropoxy)buten(2).

    4) 1,4-Bis(äthoxy)buten(2).

    5) 1,4,-Bis(trimethylsiloxy)buten(2).

**Vergleiche**

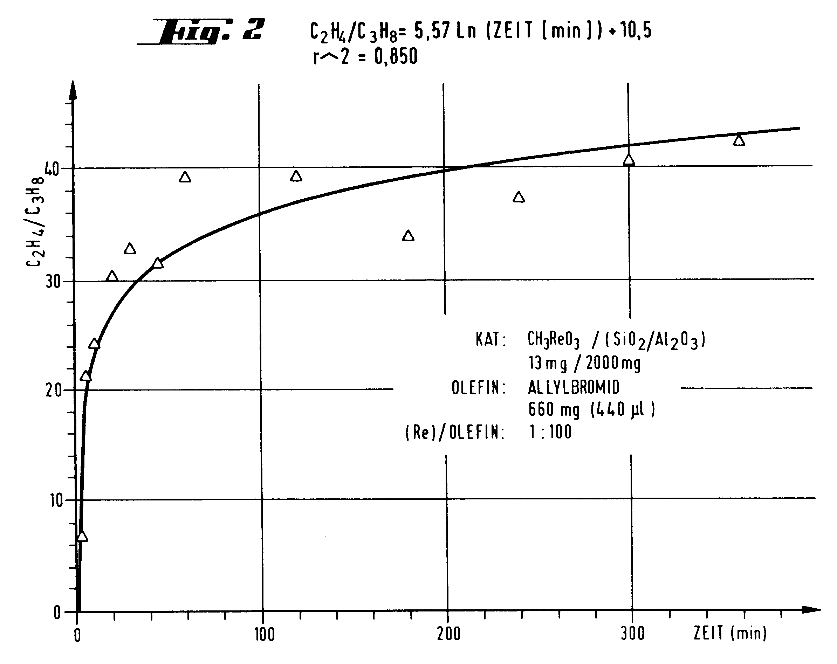

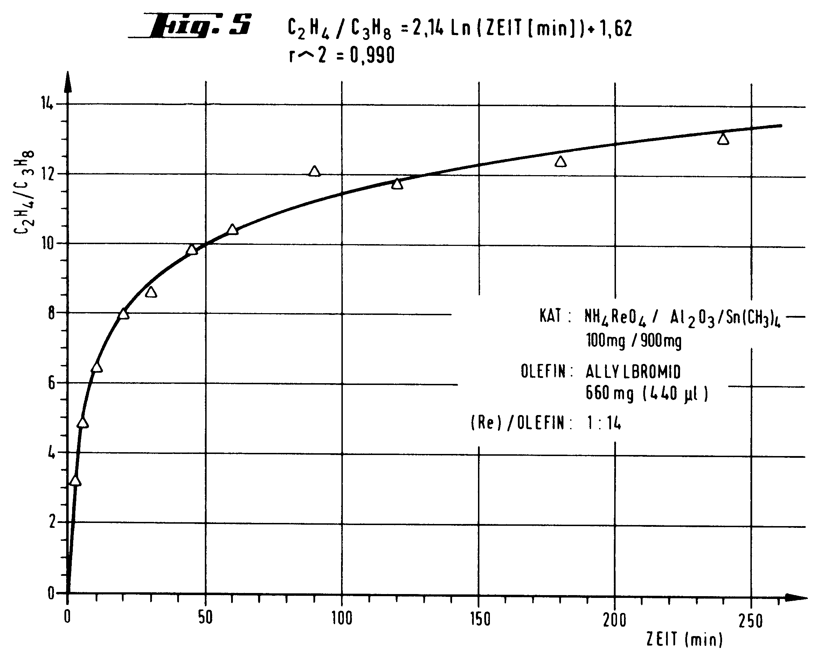

In einem mit einem Septum verschlossenen 30 ml−Reaktionsgefäß wurde unter Rühren eine Lösung von 13 mg (0,052 mmol) Methylrheniumtrioxid $CH_3ReO_3$ in 0,5 ml Dichlormethan in eine Suspension von 1000 mg Katalysatorträger ($SiO_2/Al_2O_3$ (Gewichtsverhältnis 87:13, Korngröße unter 15 $\mu$m; 2 h auf 550° C gehalten) in 10 ml Dichlormethan (über Calciumhydrid getrocknet und unter Stickstoffatmosphäre aufbewahrt) eingetragen. 0,5 ml Propan wurden als interner Standard injiziert. Nach 5−minütigem Rühren wurden 5 mmol Olefin eingespritzt (t = 0) und der Äthylen−Gehalt der Gasphase nach den aus den Figuren 1, 2, 4 und 5 ersichtlichen Zeitabständen gaschromatographisch ermittelt. Fig. 4 gibt einen Vergleich zu Fig. 1 wieder, Fig. 2 zeigt den Effekt einer gegenüber Fig. 1 größeren Katalysatorverdünnung, und Fig. 5 zeigt einen Vergleich mit dem bisher verwendeten, literaturbekannten rheniumhaltigen Katalysatorsystem nach der Vorschrift von Warwel et al. l.c.

Dazu wurden 900 mg neutrales Aluminiumoxid zunächst mit einer Lösung von 100 mg Ammonium−perrhenat $NH_4[ReO_4]$ in 10 ml Dioxan/Wasser (9 + 1 Vol.−Teile) imprägniert. Das Katalysatorsystem wurde dann bei 550°C 2 h im Sauerstoffstrom und danach 2 h im Stickstoffstrom aktiviert. Die Kinetikversuche verliefen analog denen mit Methylrheniumtrioxid $CH_3ReO_3$, doch mußten zur Aktivierung 5 min vor der Olefinzugabe 0,005 ml Tetramethylzinn $Sn(CH_3)_4$ injiziert werden, um überhaupt einen katalytischen Effekt zu erreichen.

Einen direkten Vergleich lieferte der Vergleichsversuch V1, bei dem 14 mg Ammoniumperrhenat $NH_4-[ReO_4]$ auf 1 g $SiO_2/Al_2O_3$ aufgezogen und wie zuvor angegeben im $O_2$ und $N_2$−Strom aktiviert wurden.

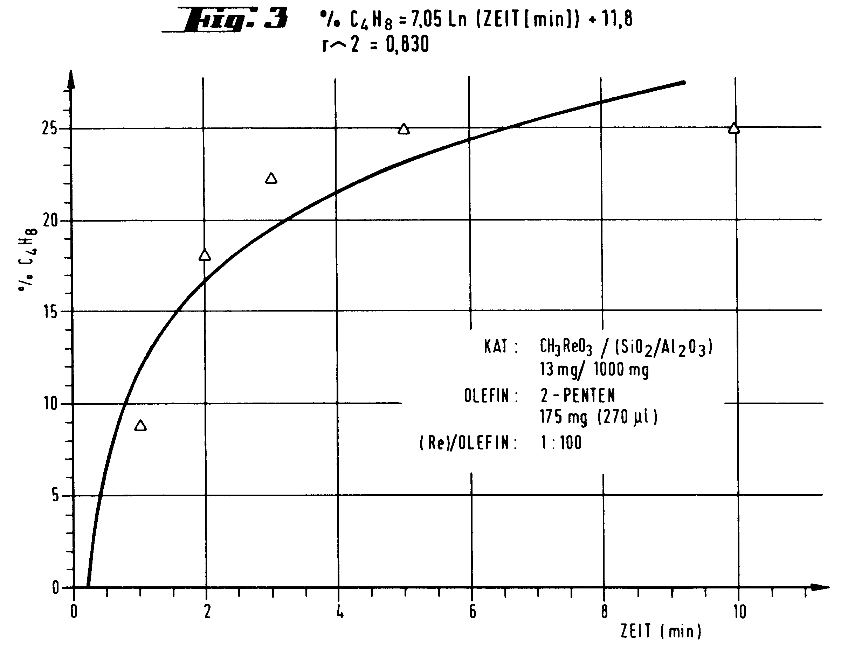

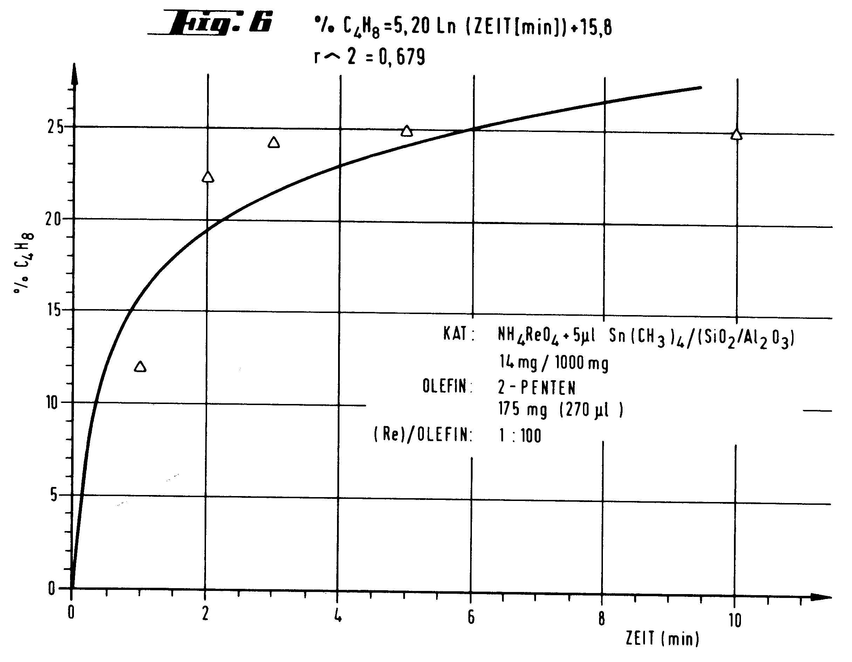

Bei der Metathese nichtfunktionalisierter Olefine erreichten $CH_3ReO_3$ einerseits und $NH_4[ReO_4]$ andererseits auf $SiO_2/Al_2O_3$ vergleichbare Aktivitäten. Die Gleichgewichts – Produktverteilung bei der Metathese eines Gemisches von 50 Mol – % 2 – Penten + 25 Mol – % 2 – Buten + 25 Mol – % 3 – Hexen stellte sich bei beiden Systemen nach ca. 5 min ein, bei $NH_4[ReO_4]$ jedoch nur nach zusätzlicher Aktivierung mit Tetramethylzinn, wie sich aus einem Vergleich von Fig. 3 und Fig. 6 ergibt.

**Effekte durch den Katalysatorträger**

Wie insbesondere die Ansätze gemäß Fig. 1 und Fig. 2 der Metathese von Allylbromid zeigen, nimmt die Aktivität des Katalysators $CH_3ReO_3$ mit der Verdünnung (in bezug auf die Menge des oxidischen Trägermaterials) stark zu:
Bei 300 min Reaktionszeit erreicht man
für 13 mg $CH_3ReO_3$ + 1000 mg $SiO_2/Al_2O_3$ den Wert Q = 30,
für 13 mg $CH_3ReO_3$ + 2000 mg $SiO_2/Al_2O_3$ den Wert Q = 40.

**Beispiele 6 – 9 – Metathese mit $(CH_3)_6Re_2O_3$**

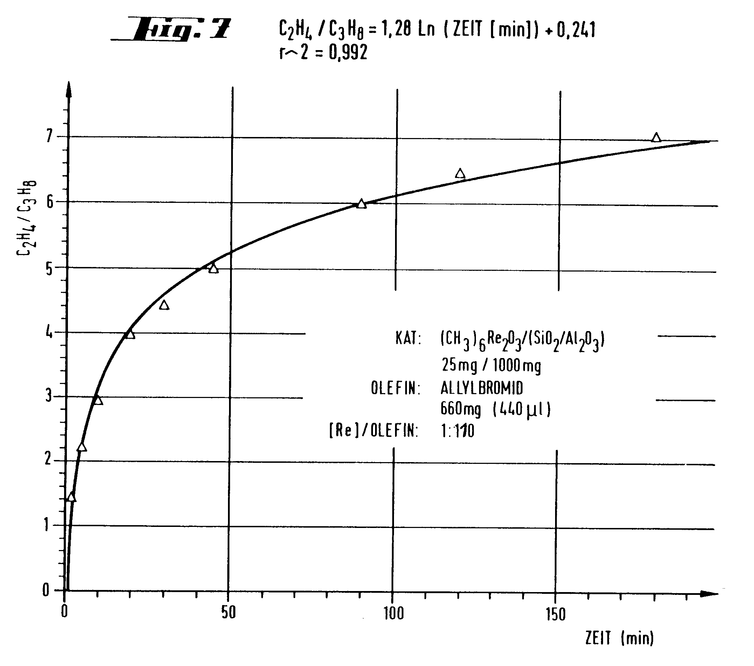

6) In einem mit einem Septum verschlossenen 30 ml – Reaktionsgefäß wurde unter Rühren eine Lösung von 25,5 mg (0,052 mmol) Hexamethyltrioxodirhenium $(CH_3)_6Re_2O_3$ in 0,5 ml Dichlormethan in eine Suspension von 1000 mg Katalysatorträger ($SiO_2/Al_2O_3$; Gewichtsverhältnis 87:13, Korngröße unter 15 $\mu$m; 2 h auf 550˚C gehalten) in 10 ml Dichlormethan (über Calciumhydrid getrocknet und unter Stickstoffatmosphäre aufbewahrt) eingetragen. 0,5 ml Propan wurden als interner Standard injiziert. Nach 5 – minütigem Rühren wurden 5 mmol Allylbromid eingespritzt (t = 0) und der Äthylen – Gehalt der Gasphase nach den aus der Fig. 7 ersichtlichen Zeitabständen gaschromatographisch ermittelt.

7 – 9) In einem 30 ml – Reaktionsgefäß mit Septum, Rückflußkühler und Quecksilberüberdruckventil wurde unter Rühren eine Lösung von 25,5 mg (0,052 mmol) Hexamethyltrioxodirhenium $(CH_3)_6Re_2O_3$ in 0,5 ml Dichlormethan in eine Suspension von 1 000 mg Katalysatorträger [$SiO_2/Al_2O_3$ (87 : 13), Korngröße unter 15 $\mu$m (Präparat Nr. 14 – 7150 der Fa. Strem Chemicals, Newburyport/ Mass. 01950 (USA), 2 h auf 550˚C erhitzt] in 10 ml Dichlormethan (über Calciumhydrid getrocknet und unter Stickstoffatmosphäre aufbewahrt) eingetragen. Der Kolbeninhalt wurde zum Sieden erhitzt. Nach Einstellen des thermischen Gleichgewichts wurden 5,2 mmol Olefin mit einer Spritze durch das Septum injiziert. Zur Isolierung des Produkts wurde nach mehrstündigem Kochen unter Rückfluß (Tab. 2) der Kontakt auf einer Fritte abgesaugt und zweimal mit je 10 ml Dichlormethan ausgewaschen. Das Lösungsmittel wurde im Vakuum einer Ölpumpe abgezogen, das Produkt ausgewogen und durch einen Gaschromatographen mit angekoppeltem Massenspektrometer auf Reinheit überprüft.

## Tabelle 2

| Beispiel/Olefin (*) | Zeit [h] | Menge [mg] | Auswaage [mg] | Reinheit [%] | Ausbeute [%] |
|---|---|---|---|---|---|
| 7) Allylbromid | 20 | 680 | 72 | 98 | 12 |
| 8) Undecensäureäthylester | 20 | 1410 | 840 | 57 | 34 |
| 9) Allyltrimethyl-silyläther | 15 | 705 | 550 | 48 | 41 |

(*) Metatheseprodukte:

    7) 1,4-Dibrombuten(2).

    8) Eicosen(10)-1,1'-dicarbonsäurediäthylester.

    9) 1,4-Bis(trimethylsiloxy)buten(2).

**Beispiel 10 — Metathese mit $(CH_3)_4 - Re_2O_4$**

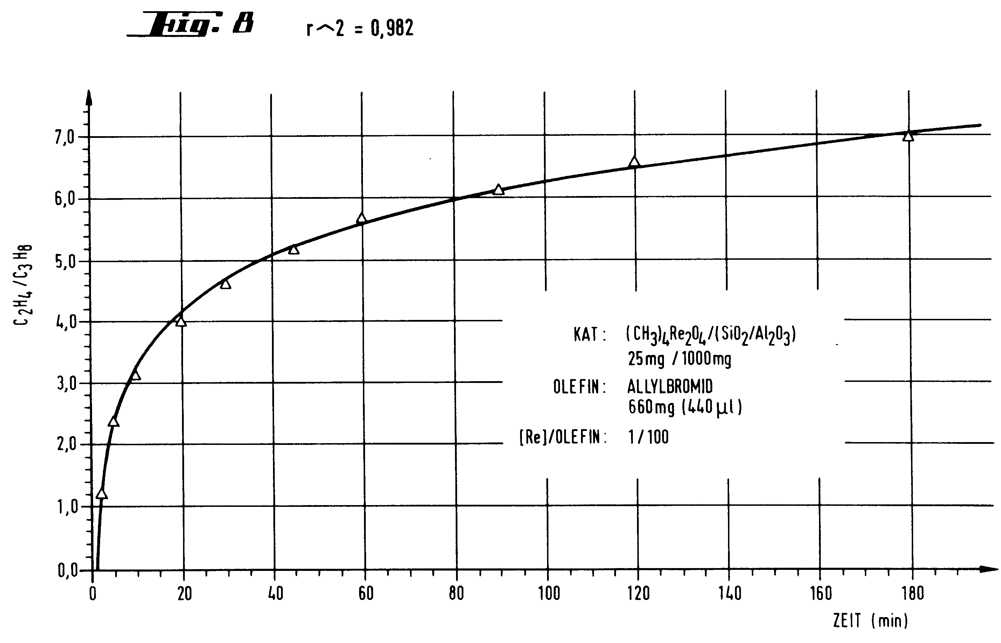

In einem mit einem Septum verschlossenen 30 ml-Reaktionsgefäß wurde unter Rühren eine Lösung von 25,8 mg (0,052 mmol) Tetramethyltetraoxodirhenium $(CH_3)_4 Re_2O_4$ in 0,5 ml Dichlormethan in eine Suspension von 1000 mg Katalysatorträger $(SiO_2/Al_2O_3$; Gewichtsverhältnis 87:13, Korngröße unter 15 $\mu$m; 2 h auf 550° C gehalten) in 10 ml Dichlormethan (über Calciumhydrid getrocknet und unter Stickstoffatmosphäre aufbewahrt) eingetragen. 0,5 ml Propan wurden als interner Standard injiziert. Nach 5-minütigem Rühren wurden 5 mmol Allylbromid eingespritzt (t = 0) und der Äthylen-Gehalt der Gasphase nach den aus der Fig. 8 ersichtlichen Zeitabständen gaschromatographisch ermittelt.

**Beispiele 11—14 — Verbindungen der Formel $R^1{}_a Re_b O_c$**

**11. Trimethyl(oxo)rhenium, $(CH_3)_3 ReO$**

a) Eine Lösung von 4,14 g (10 mmol) der Verbindung $[(CH_3)_3 SnO]ReO_3$ in 100 ml wasserfreiem Tetrahydrofuran wurde rasch nacheinander bei Raumtemperatur mit 2,62 g (10 mmol) Triphenylphosphin $P(C_6H_5)_3$ und mit 10,85 g (0,1 mol) Trimethylchlorsilan $(CH_3)_3 SiCl$ unter kräftigem magnetischem Rühren versetzt. Nach 3 Stunden Rühren bei Raumtemperatur hatte sich ein grüner Bodensatz gebildet, der aus der Verbindung $ReOCl_3[OP(C_6H_5)](OC_4H_8)$ bestand $(OC_4H_8$ = Tetrahydrofuran). Abfiltrieren dieses Niederschlags von der überstehenden Lösung, Waschen mit Tetrahydrofuran und Trocknen im Vakuum einer Ölpumpe ergab 6,19 g (94 % d. Th.) dieser Verbindung in reiner Form.

b) 3,29 g (5 mmol) der unter a) hergestellten Verbindung wurden in 20 ml Tetrahydrofuran suspendiert. Zu dieser Suspension gab man tropfenweise bei 0° C eine 0,2-molare Lösung von Lithiummethyl $LiCH_3$ (insgesamt 16 mmol) und ließ das resultierende Reaktionsgemisch noch 3 Stunden bei Raumtemperatur rühren. Die flüchtigen Anteile wurden dann im Vakuum einer Ölpumpe entfernt. Der dunkelbraun gefärbte, meist etwas ölige Rückstand wurde mit ca. 10 ml Toluol extrahiert. Der Extrakt wurde im Vakuum einer Ölpumpe zur Trockne gebracht. Der Rückstand wurde bei $-60°$ C an einer Säule (40 cm lang, 2 cm Durchmesser) von silanisiertem, ausgeglühtem Kieselgel (Fa. Merck) chromatographiert, wobei sich mit Toluol die gelbe Zone der Verbindung $(CH_3)_3 ReO$ entwickelte. Das Eluat wurde im Vakuum einer Ölpumpe eingeengt. Der Rückstand wurde dann in 20 ml n-Pentan gelöst, und die Lösung wurde bei $-80$ bis $-40°$ C der Kristallisation überlassen. Es ergaben sich 198 mg (16 % d.Th.) gelbe Nadeln von $(CH_3)_3 ReO$. — Charakterisierung: Löslich in den gebräuchlichen, wasserfreien organischen Lösungsmitteln, insbesondere Methylenchlorid, Tetrahydrofuran und Toluol. Die Substanz ist fest

7

und in Lösung allerdings nur bei Lagerung im Temperaturbereich $-80$ bis $-40°C$ einige Zeit unzersetzt haltbar. $-$ IR (cm$^{-1}$, KBr): 978 sst [v(Re = O)]. $-$ $^1$H$-$NMR (270 MHz; 28$°$C, CD$_2$Cl$_2$): $\delta$CH$_3$ = 5,41 (Singulett). $-$ FD$-$Massenspektrum: Molekül$-$Ion bei m/z = 248 ($^{187}$Re), mit korrektem Isotropenmuster $^{185}$Re/$^{187}$Re.

## 12. Tris(neopentyl)oxorhenium, Re[CH$_2$C(CH$_3$)$_3$]$_3$O

Diese Verbindung wurde analog der Vorschrift gemäß Beispiel 11 synthetisiert, wobei man statt Lithiummethyl das Lithiumneopentyl Li[CH$_2$C(CH$_3$)$_3$] einsetzte. Das Produkt wurde analog aufgearbeitet. Ausbeute 249 mg (12 % d. Theorie). $-$ Charakterisierung: Gelbe Kristalle; über einen längeren Zeitraum (ca. 8 Tage) unzersetzt haltbar nur bei Temperaturen unterhalb $-20°C$. $-$ IR (cm$^{-1}$, KBr): 975 cm$^{-1}$ sst [v(Re = O)]. $-$ $^1$H$-$NMR (400 MHz, 28$°$C, CDCl$_3$): $\delta$CH$_3$ = 1,12 (Singulett, 9H), $\delta$CH$_2$ = 2,21 [etwas verbreitertes Singulett, 2H]. $-$ FD$-$Massenspektrum: Molekül$-$Ion bei m/z = 416 ($^{187}$Re) mit korrektem Isotopenmuster $^{185}$Re/$^{187}$Re.

## 13. 2,2,2$-$Trifluoräthyl)trioxorhenium, (CF$_3$CH$_2$)ReO$_3$

Eine Lösung von 4,84 g (10 mmol) Dirheniumheptoxid Re$_2$O$_7$ (Fa. Degussa, 76,9 % Re) in 100 ml eines wasserfreien Lösungsmittels wie Tetrahydrofuran wurde bei Raumtemperatur mit 20 mmol einer 0,5$-$molaren Lösung von Bis(2,2,2$-$Trifluoräthyl)zink Zn(CH$_2$CF$_3$)$_2$ in Tetrahydrofuran binnen 10 min tropfen$-$weise versetzt und dann noch 80 min bei Raumtemperatur gerührt. Die flüchtigen Anteile wurden dann im Vakuum einer Ölpumpe in eine Kühlfalle abgezogen, die mit flüssigem Stickstoff gekühlt war. Der Rückstand wurde in einem Hochvakuum bei 40 $-$ 85$°$C an einem wassergekühlten Sublimationsfinger sublimiert. Dabei erhielt man 2,41 g (38 % d. Theorie) schwach gelbliche, nadelförmige Kristalle der Verbindung (CF$_3$CH$_2$)ReO$_3$. Die Synthese gelingt auch mit nicht$-$sublimiertem Re$_2$O$_7$, doch sind die Ausbeuten dann geringer (Bereich 10 $-$ 18 %).

Die Substanz ist folgendermaßen charakterisiert: Löslich in den meisten wasserfreien organischen Lösungsmitteln, insbesondere in Tetrahydrofuran, Methylenchlorid und Toluol, wobei sich nahezu farblose, lichtempfindliche Lösungen bilden. IR (cm$^{-1}$, KBr): 1048 st, 960 sst [v(Re = O)]. $-$ $^1$H$-$NMR (CDCl$_3$, 28$°$C): $\delta$(CH$_2$) = 2,30 [Quartett]. $^{17}$O$-$NMR (CDCl$_3$, 28$°$C): $\delta$(O) = 590 ppm. $-$ EI$-$MS: m/z = 318 (Molekül$-$Ion mit korrektem Isotopenmuster $^{185}$RE/$^{187}$Re). Die Substanz kann bei Eistemperatur (0$°$C) unzersetzt aufbewahrt werden, wenn für Lichtausschluß gesorgt ist. $-$ Elementaranalyse: Ber. für C$_2$H$_2$F$_3$O$_3$Re (317,21): C 7,57, H 0,63, F 17,97, Re 58,70; Gef.: C 7,60, H 0,70, F 18,00, Re 58,59.

Dieselbe Verbindung erhielt man in 40 $-$ 48 % Ausbeute, wenn man Dirheniumheptoxid Re$_2$O$_7$ mit der äquimolaren Menge Tetrakis(2,2,2,$-$trifluoräthyl)zinn Sn(CH$_2$CF$_3$)$_4$ unter den in der vorgenannten Arbeits$-$vorschrift genannten Bedingungen umsetzte, wobei nur der Unterschied bestand, daß die Komponenten nicht bei Raumtemperatur, sondern in siedendem Tetrahydrofuran 1 bis 2 h miteinander umgesetzt wurden. Die Aufarbeitung des Produktes erfolgte analog.

### Verwendung des (CF$_3$CH$_2$)ReO$_3$

Bei der Anwendung dieser Verbindung als Katalysator für die Olefin$-$Metathese wird ebenso verfahren wie es in den Beispielen 1 bis 5 für Methylrheniumtrioxid CH$_3$ReO$_3$ beschrieben ist.

## 14. (2,3,4,5,6$-$Pentafluorphenylmethyl)trioxorhenium, C$_6$F$_5$CH$_2$ReO$_3$

Aus einer Lösung von 6,16 g (20 mmol) 2,3,4,5,6$-$Pentafluorphenyl)methyljodid C$_6$F$_5$CH$_2$J wurde zunächst nach bekanntem Verfahren durch Umsetzung mit Magnesiumspänen in Diäthyläther als Lö$-$sungsmittels die entsprechende Grignard$-$Verbindung C$_6$F$_5$CH$_2$MgJ hergestellt. Die erhaltene Lösung wurde dann mit 12,3 g (9 mmol) wasserfreiem Zinkchlorid versetzt. Die entstandene Reaktionsmischung kochte man dann 5 h unter Rückfluß. Die so erzielbare Verbindung Bis(2,3,4,5,6$-$pentafluorphenylmethyl)$-$zink Zn(CH$_2$C$_6$F$_5$)$_2$ konnte aus der Lösung nach Filtration durch Umkristallisation bei tiefen Temperaturen ($-80$ bis $-40°C$) erhalten werden. 2,14 g (5 mmol) Bis(2,3,4,5,6$-$pentafluorphenylmethyl)zink Zn$-$(CH$_2$C$_6$F$_5$)$_2$ wurden bei Raumtemperatur zu einer Lösung von 4,84 g (10 mmol) Dirheniumheptoxid Re$_2$O$_7$ (Fa. Degussa, 76,9 % Re) in 100 ml eines wasserfreien Lösungsmittels wie Tetrahydrofuran gegeben. Die resultierende Lösung wurde noch 60 min bei Raumtemperatur gerührt. Die flüchtigen Anteile wurden dann im Vakuum einer Ölpumpe in eine Kühlfalle abgezogen, die mit flüssiger Luft gekühlt war. Der Rückstand wurde dreimal mit jeweils 20 ml wasserfreiem Toluol extrahiert, der Extrakt filtriert und das Filtrat dann im Vakuum einer Ölpumpe bei 0°C zur Trockne gebracht. Der Rückstand wurde aus einem Lösungsmittelge − misch, das aus gleichen Volumina n − Hexan und Toluol bestand, bei − 40 bis − 8°C umkristallisiert. Dabei erhielt man 581 mg (28 % d. Theorie, bezogen auf eingesetzte Zink − Verbindung) schwach gelb gefärbte oktaedrische Kristalle des Katalysators $(C_6F_5CH_2)ReO_3$.

Die Substanz ist folgendermaßen charakterisiert: Löslich in den meisten gebräuchlichen wasserfreien organischen Lösungsmitteln, insbesondere Toluol, Methylenchlorid und Tetrahydrofuran. − $\underline{IR}$ ($cm^{-1}$, KBr): 1058 st, 958 sst $[v(Re = O)]$. − $^1H − NMR$ ($CDCl_3$, 28°C): $\delta(CH_2)$ = 2,39 ppm etwas verbreitertes Singulett]. − $^{17}O − NMR$ ($CDCl_3$, 28°C): $\delta(O)$ = 604 ppm. − $\underline{EI − MS}$: m/z = 416 (Molekül − Ion mit korrektem Isotopenmuster $^{185}Re/^{187}Re$). − Elementaranalyse: Ber. für $C_6F_5CH_2ReO_3$ (415,26): C 20,25, H 0,48, F 22,87, Re 44,84; Gef. C 20,20, H 0,50, F 22,60, Re 45,00.

## Patentansprüche

1. Verbindungen der allgemeinen Formel $R^1_aRe_bO_c$ (I), worin a 1 bis 6, b 1 bis 4 und c 1 bis 14 sind und die Summe von a, b und c so ist, daß sie der 5 − bis 7 − Wertigkeit des Rheniums gerecht wird mit der Maßgabe, daß c nicht größer als 3•b ist, und worin $R^1$ einen Alkylrest mit 1 bis 9 C − Atomen, einen Cycloalkylrest mit 5 bis 10 C − Atomen oder einen Aralkylrest mit 7 bis 9 C − Atomen darstellt, wobei $R^1$ wenigstens teilweise fluoriert sein kann, die Verbindungen nicht mehr als drei Gruppen mit mehr als 6 C − Atomen je Rheniumatom enthalten und an das C − Atom in $\alpha$ − Stellung noch wenigstens ein Wasserstoffatom gebunden ist und wobei die Verbindungen Methylrheniumtrioxid $CH_3ReO_3$, das $(CH_3)_6Re_2O_3$, das $(CH_3)_4Re_2O_4$, $(CH_3)_4ReO$, das $(CH_3)_3ReO_2$, das $[(CH_3)_3C − CH_2]_3ReO_2$, das $[ − (CCH_3)_3C − CH_2]_4Re_2O_4$ und das Pentamethylcyclopentadienyl − Rheniumtrioxyd $(\eta^5 − C_5(CH_3)_5)ReO_3$ ausgenommen sind.

2. Verwendung von Verbindungen der allgemeinen Formel $R^1_aRe_bO_c$ (I), worin a 1 bis 6, b 1 bis 4 und c 1 bis 14 sind und die Summe von a, b und c so ist, daß sie der 5 − bis 7 − Wertigkeit des Rheniums gerecht wird mit der Maßgabe, daß c nicht größer als 3•b ist, und worin $R^1$ einen Alkylrest mit 1 bis 9 C − Atomen, einen Cycloalkylrest mit 5 bis 10 C − Atomen oder einen Aralkylrest mit 7 bis 9 C − Atomen darstellt, wobei $R^1$ wenigstens teilweise fluoriert sein kann, die Verbindungen nicht mehr als drei Gruppen mit mehr als 6 C − Atomen je Rheniumatom enthalten und an das C − Atom in $\alpha$ − Stellung noch wenigstens ein Wasserstoffatom gebunden ist, die auf oxydische Trägermaterialien aufgebracht sind, als Katalysatoren zur Metathese olefinischer Verbindungen.

3. Verfahren zur Herstellung von metallorganischen Katalysatoren, wie im Anspruch 2 definiert, dadurch gekennzeichnet, daß man die Rheniumverbindung aus einem wasserfreien, gegenüber den Rhenium − verbindungen inerten Lösungsmittel auf den Katalysatorträger aufbringt und die flüchtigen Anteile entfernt.

4. Verfahren zur Metathese vor Olefinen, dadurch gekennzeichnet, daß man Olefine des Typs $YCZ = CZ − (CX_2)_nR^2$ (II), worin die Gruppen YCZ und $CZ − (CX_2)_nR^2$ verschieden sind und n eine ganze Zahl von 1 bis 28, X H oder F ist, Y H oder Alkyl mit 1 bis 10 C − Atomen ist und Z H oder einen nicht − aromatischen Kohlenwasserstoffrest mit 1 bis 6 C − Atomen darstellt und der Substituent $R^2$ H, Alkyl, Halogen, $COOR^3$ oder $OR^4$ ist, worin $R^3$ und $R^4$ Alkyl mit 1 bis 15, vorzugsweise 1 bis 6, Kohlenstoff − atomen oder Phenyl ist, das am Ring noch 1 bis 3 Substituenten enthalten kann oder worin $R^4$ Trialkylsilyl $R_3^5Si$ ist, worin $R^5$ Alkyl mit 1 bis 5, vorzugsweise 1 bis 3, C − Atomen darstellt, an Katalysatoren umsetzt, die aus oxydischen Trägermaterialien bestehen, auf die Verbindungen der im Anspruch 2 definierten Art aufgebracht sind.

5. Verfahren nach Anspruch 4, dadurch gekennzeichnet, daß man ein funktionalisiertes Olefin der Metathese − Reaktion unterwirft.

6. Verfahren nach Anspruch 4 oder 5, dadurch gekennzeichnet, daß man ein Cycloolefin der Metathese − Reaktion unterwirft.

7. Verfahren nach einem oder, mehreren der Ansprüche 4 bis 6, dadurch gekennzeichnet, daß man in Abwesenheit eines Aktivators arbeitet.

**8.** Verfahren nach einem oder mehreren der Ansprüche 1 bis 7, dadurch gekennzeichnet, daß der Katalysatorträger aus Aluminiumoxid oder Kombinationen davon mit Siliziumdioxid besteht und durch Ausglühen getrocknet ist.

**9.** Verfahren nach einem oder mehreren der Ansprüche 4 bis 8, dadurch gekennzeichnet, daß der Katalysator als wirksame Substanz Methylrheniumtrioxid enthält.

**10.** Verfahren nach einem oder mehreren der Ansprüche 4 bis 9, dadurch gekennzeichnet, daß man im Temperaturbereich von 0 bis 60˚C, vorzugsweise von 10 bis 30˚C und bei Atmosphärendruck, arbeitet.

**11.** Verbindungen nach Anspruch 1, dadurch gekennzeichnet, daß $R^1$ teilweise fluoriert ist.

**12.** Verbindungen der Formel $R^1{}_a Re_b O_c$ (I) nach Anspruch 1 oder 11, dadurch gekennzeichnet, daß $a = 3$, $b = 1$ und $c = 1$ ist.

**Claims**

**1.** A compound of the formula $R^1{}_a Re_b O_c$ (I) in which a is from 1 to 6, b is from 1 to 4 and c is from 1 to 14, and sum of a, b and c is such that it satisfies the pentavalency to heptavalency of the rhenium, with the proviso that c is not greater than $3 \cdot b$, and in which $R^1$ is an alkyl radical having 1 to 9 carbon atoms, a cycloalkyl radical having 5 to 10 carbon atoms or an aralkyl radical having 7 to 9 carbon atoms, where $R^1$ may be at least partially fluorinated, the compounds contain not more than three groups containing more than 6 carbon atoms per rhenium atom, and at least one hydrogen atom is bonded to the carbon atom in the $\alpha$−position, and where the compounds methylrhenium trioxide $CH_3 ReO_3$, $(CH_3)_6 Re_2 O_3$, $(CH_3)_4 Re_2 O_4$, $(CH_3)_4 ReO,(CH_3)_3 ReO_2$, $[(CH_3)_3 C - CH_2]_3 ReO_2$, $[CCH_3)_3 C - CH_2]_4 Re_2 O_4$ and pentamethylcyclopentadienylrhenium trioxide $(\eta^5 - C_5 (CH_3)_5)ReO_3$ are excluded.

**2.** The use of a compound of the formula $R^1{}_a Re_b O_c$ (I) in which a is from 1 to 6, b is from 1 to 4 and c is from 1 to 14, and sum of a, b and c is such that it satisfies the pentavalency to heptavalency of the rhenium, with the proviso that c is not greater than $3 \cdot b$, and in which $R^1$ is an alkyl radical having 1 to 9 carbon atoms, a cycloalkyl radical having 5 to 10 carbon atoms or an aralkyl radical having 7 to 9 carbon atoms, where $R^1$ may be at least partially fluorinated, the compounds contain not more than three groups containing more than 6 carbon atoms per rhenium atom, and at least one hydrogen atom is bonded to the carbon atom in the $\alpha$−position, which have been applied to oxidic support materials, as catalysts for the metathesis of olefinic compounds.

**3.** A process for the preparation of organometallic catalysts as defined in claim 2, which comprises applying the rhenium compound to the catalyst support from an anhydrous solvent which is inert toward the rhenium compounds, and removing the volatile constituents.

**4.** A process for the metathesis of olefins, which comprises reacting olefins of the $YCZ = CZ - (CX_2)_n R^2$ (II) type, in which the groups YCZ and $CZ - (CX_2)_n R^2$ are different and n is an integer from 1 to 28, X is H or F, Y is H or alkyl having 1 to 10 carbon atoms, and Z is H or a non−aromatic hydrocarbon radical having 1 to 6 carbon atoms, and the substituent $R^2$ is H, alkyl, halogen, $COOR^3$ or $OR^4$ in which $R^3$ and $R^4$ are alkyl having 1 to 15 carbon atoms, preferably 1 to 6 carbon atoms, or phenyl, which may also contain 1 to 3 substituents on the ring, or in which $R^4$ is trialkylsilyl, $R^5{}_3 Si$ in which $R^5$ is alkyl having 1 to 5 carbon atoms, preferably 1 to 3 carbon atoms, on catalysts comprising oxidic support materials which have been applied to the compounds of the type defined in claim 2.

**5.** The process as claimed in claim 4, wherein a functionalised olefin is subjected to the metathesis reaction.

**6.** The process as claimed in claim 4 or 5, wherein a cycloolefin is subjected to the metathesis reaction.

**7.** The process as claimed in one or more of claims 4 to 6, carried out in the absence of an activator.

EP 0 373 488 B1

**8.** The process as claimed in one or more of claims 1 to 7, wherein the catalyst support comprises aluminum oxide or combinations thereof with silicon dioxide and has been dried by ignition.

**9.** The process as claimed in one or more of claims 4 to 8, wherein the catalyst contains methyl rhenium trioxide as the active substance.

**10.** The process as claimed in one or more of claims 4 to 9, carried out in the temperature range from 0 to 60°C, preferably from 10 to 30°C, and at atmospheric pressure.

**11.** A compound as claimed in claim 1, wherein $R^1$ is partially fluorinated.

**12.** A compound of the formula $R^1_a Re_b O_c$ (I) as claimed in claim 1 or 11, wherein a = 3, b = 1 and c = 1.

**Revendications**

**1.** Composés de formule générale $R^1_a Re_b O_c$ (I), dans laquelle a vaut de 1 à 6, b vaut de 1 à 4 et c vaut de 1 à 14, et la somme de a, b et c est telle qu'elle prend en compte le caractère pentavalent à heptavalent du rhénium, du moment que c n'est pas supérieur à 3•b, et où $R^1$ est un radical alkyle ayant de 1 à 9 atomes de carbone, un radical cycloalkyle ayant de 5 à 10 atomes de carbone ou un radical aralkyle ayant de 7 à 9 atomes de carbone, $R^1$ pouvant être au moins partiellement fluoré, les composés ne contiennent pas plus de trois groupes ayant plus de 6 atomes de carbone par atome de rhénium, et encore au moins un atome d'hydrogène est lié à l'atome de carbone en position $\alpha$, et ce à l'exception des composés suivants : le trioxyde de méthyl-rhénium $CH_3 ReO_3$, le $(CH_3)_6 Re_2 O_3$, le $(CH_3)_4 Re_2 O_4$, le $(CH_3)_4 ReO$, le $(CH_3)_3 ReO_2$, le $[(CH_3)_3 C - CH_2]_3 ReO_2$, le $[(CH_3)_3 C - CH_2]_4 Re_2 O_4$, ainsi que le trioxyde de pentaméthylcyclopentadiényl-rhénium $(\eta^5 - C_5 (CH_3)_5) ReO_3$.

**2.** Utilisation de composés de formule générale $R^1_a Re_b O_c$ (I), dans laquelle a vaut de 1 à 6, b vaut de 1 à 4 et c vaut de 1 à 14, et la somme de a, b et c est telle qu'elle prend en compte le caractère pentavalent à heptavalent du rhénium, du moment que c n est pas supérieur à 3•b, et où $R^1$ est un radical alkyle ayant de 1 à 9 atomes de carbone, un radical cycloalkyle ayant de 5 à 10 atomes de carbone ou un radical aralkyle ayant de 7 à 9 atomes de carbone, $R^1$ pouvant être au moins partiellement fluoré, les composés ne contiennent pas plus de trois groupes ayant plus de 6 atomes de carbone par atome de rhénium, et encore au moins un atome d'hydrogène est lié à l'atome de carbone en position $\alpha$, qui sont appliqués sur des matériaux supports à base d'oxydes, en tant que catalyseurs pour la métathèse de composés oléfiniques.

**3.** Procédé pour préparer des catalyseurs organométalliques selon la revendication 2, caractérisé en ce qu'on applique sur le support de catalyseur le composé du rhénium à partir d'un solvant anhydre, inerte vis-à-vis des composés du rhénium, et que l'on élimine les fractions volatiles.

**4.** Procédé pour métathétiser des oléfines, caractérisé en ce qu'on fait réagir des oléfines du type $YCZ = CZ - (CX_2)_n R^2$ (II), où les groupes YCZ et $CZ - (CX_2)_n R^2$ sont différents, et n est un nombre entier de 1 à 28, X est H ou F, Y est H ou un radical alkyle ayant de 1 à 10 atomes de carbone, et Z est H ou un radical hydrocarboné non aromatique ayant de 1 à 6 atomes de carbone, et le substituant $R^2$ est H ou un radical alkyle, halogéno, $COOR^3$ ou $OR^4$, où $R^3$ et $R^4$ sont chacun un radical alkyle ayant de 1 à 15, de préférence 1 à 6 atomes de carbone, ou le radical phényle, qui peut encore contenir sur le noyau 1 à 3 substituants, ou encore où $R^4$ est un radical trialkylsilyle $R^5_3 Si$, où $R^5$ est un radical alkyle ayant de 1 à 5 et de préférence de 1 à 3 atomes de carbone, sur des catalyseurs qui sont constitués de matériaux supports à base d'oxydes appliqués sur les composés du type selon la revendication 2.

**5.** Procédé selon la revendication 4, caractérisé en ce qu'on soumet à la réaction de métathèse une oléfine fonctionnalisée.

**6.** Procédé selon la revendication 4 ou 5, caractérisé en ce qu'on soumet à la réaction de métathèse une cyclo-oléfine.

11

**7.** Procédé selon l'une ou plusieurs des revendications 4 à 6, caractérisé en ce qu'on travaille en l'absence d'activateur.

**8.** Procédé selon l'une ou plusieurs des revendications 1 à 7, caractérisé en ce que le support de catalyseur est constitué d'alumine ou de combinaisons d'alumine et de silice, et est séché par calcination.

**9.** Procédé selon l'une ou plusieurs des revendications 4 ou 8, caractérisé en ce que le catalyseur contient comme matière active du trioxyde de méthylrhénium.

**10.** Procédé selon l'une ou plusieurs des revendications 4 à 9, caractérisé en ce qu'on travaille sur un intervalle de températures de 0 à 60°C et de préférence de 10 à 30°C, et sous la pression atmosphérique.

**11.** Composés selon la revendication 1, caractérisés en ce que $R^1$ est partiellement fluoré.

**12.** Composés de formule $R^1_a Re_b O_c$ (I) selon la revendication 1 ou 11, caractérisés en ce que a = 3, b = 1 et c = 1.

**Fig. 1** $C_2H_4/C_3H_8 = 5,48$ Ln ( ZEIT [min] ) $-0,936$

$r{\char`\^}2 = 0,954$

KAT: $CH_3ReO_3 / (SiO_2/Al_2O_3)$
13 mg / 1000 mg
OLEFIN: ALLYLBROMID 660 mg (440 µl )
(Re)/OLEFIN: 1 : 100

ZEIT (min)

EP 0 373 488 B1

_Fig. 2_     $C_2H_4/C_3H_8 = 5,57 \, Ln \, (ZEIT \, [min]) + 10,5$
$r^2 = 0,850$

KAT:     $CH_3ReO_3 \, / \, (SiO_2/Al_2O_3)$
13 mg / 2000 mg
OLEFIN:     ALLYLBROMID
660 mg (440 µl)
(Re)/OLEFIN:     1 : 100

EP 0 373 488 B1

**Fig. 3** % $C_4H_8 = 7,05$ Ln (ZEIT [min]) + 11,8
$r^2 = 0,830$

KAT : $CH_3ReO_3$ / $(SiO_2/Al_2O_3)$
13 mg / 1000 mg

OLEFIN : 2 – PENTEN
175 mg (270 µl )

(Re)/OLEFIN : 1 : 100

% $C_4H_8$ (vertical axis label)

ZEIT ( min )

EP 0 373 488 B1

Fig. 4 $C_2H_4/C_3H_8 = 2,69$ Ln (ZEIT [min]) + 4,99

$r^2 = 0,962$

KAT: $NH_4ReO_4 / (SiO_2/Al_2O_3)$ 14 mg/ 1000 mg

OLEFIN: ALLYLBROMID 660 mg (440 µl)

(Re) /OLEFIN: 1:100

EP 0 373 488 B1

Fig. 5 $C_2H_4 / C_3H_8 = 2,14 \, Ln \, (ZEIT \, [min]) + 1,62$
$r^2 = 0,990$

KAT : $NH_4ReO_4 / \; Al_2O_3 / Sn(CH_3)_4$ ———
100 mg / 900 mg

OLEFIN : ALLYLBROMID
660 mg ( 440 µl )

(Re) / OLEFIN : 1 : 14

EP 0 373 488 B1

**Fig. 6**    % $C_4H_8 = 5,20$ Ln (ZEIT[min]) + 15,8

$r \frown 2 = 0,679$

KAT :    $NH_4ReO_4 + 5\mu l$   $Sn(CH_3)_4/(SiO_2/Al_2O_3)$
14 mg / 1000 mg

OLEFIN :    2 - PENTEN
175 mg (270 $\mu l$ )

(Re)/OLEFIN :    1 : 100

% $C_4H_8$

ZEIT (min)

EP 0 373 488 B1

Fig. 7

$C_2H_4/C_3H_8 = 1{,}28 \; Ln \; (ZEIT \; [min]) + 0{,}241$

$r^2 = 0{,}992$

KAT: $(CH_3)_6 Re_2 O_3 /(SiO_2/Al_2O_3)$
25mg / 1000mg

OLEFIN: ALLYLBROMID
660mg (440 µl)

[Re]/OLEFIN: 1:170

ZEIT (min)

$C_2H_4/C_3H_8$

**Fig. 8**  $r^2 = 0,982$

Ordinate: $C_2H_4 / C_3H_8$

Abscissa: ZEIT (min)

KAT: $(CH_3)_4Re_2O_4 / (SiO_2/Al_2O_3)$
25 mg / 1000 mg

OLEFIN: ALLYLBROMID
660 mg (440 µl)

[Re]/OLEFIN: 1/100